# EUROPEAN PATENT APPLICATION

(11) **EP 2 085 406 A1**
(43) Date of publication of application: **05.08.2009**
(21) Application number: 09159269.1
(22) Date of filing: 26.07.2004
(51) Int. Cl.: C07K 14/62, C07K 14/765, A61P 3/10, C07K 1/113, A61K 38/28

(54) **Long lasting insulin derivatives and methods thereof**

(30) Priority: 25.07.2003 US 490110 P; 25.03.2004 US 556585 P
(62) Divisional of application: 04801809.7
(71) Applicant: ConjuChem Biotechnologies Inc., Montreal, QC H2X 3Y8 (CA)
(72) Inventor: Bridon, Dominique P., San Francisco, CA 94114 (US); Castaigne, Jean-Paul, Town of Mount Royal Québec H3P 1Y6 (CA); Huang, Xicai, Kirkland Québec H9J 3X2 (CA); Leger, Roger, Saint-Lambert Québec J4R 2V8 (CA); Robitaille, Martin, Granby Québec J2G 6A2 (CA)
(74) Representative: Williams, Gareth Owen

(57) **Abstract**

The present invention relates to an insulin derivative comprising an insulin molecule and a reactive group for covalently bonding a blood component, wherein preferably the insulin molecule is human natural insulin molecule and the reactive group is coupled to an amino acid of the insulin molecule at a position selected from the positions Gly A1, Phe B1 and Lys B29.

## Description

### BACKGROUND OF THE INVENTION

### (a) Field of the Invention

This invention relates to a long lasting insulin derivative. More particularly, the insulin derivative comprises an insulin molecule and a reactive group coupled thereto, the reactive group being for covalently bonding a blood component hence generating a long lasting insulin derivative.

### (b) Description of Prior Art

Insulin is a vital endocrine hormone that binds to a cellular surface receptor setting off a cascade of events culminating in glucose absorption from the blood. Impaired levels of insulin lead to severe disorders such as types I and II diabetes. Type I diabetes is a life threatening disease where the patient must daily self-administer multiple doses insulin for survival. Type II diabetes, is also a severe medical disease where the endogenous levels of insulin can no longer maintained correct levels of glycemia because the patient due to a tolerance developed by the patient to endogenous levels of insulin. In order to reduce the onset of long-term consequences, a treatment with insulin becomes necessary after failure in lifestyle changes or when traditional glycemia controlling drugs become ineffective.

Success in the control of glycaemic disorder is highly related with the compliance of patients to the treatment, and reducing the frequency of injection needed is desirable. To do so, it would be highly desirable to be provided with a new long lasting insulin derivative.

### SUMMARY OF THE INVENTION

In accordance with the present invention there is provided an insulin derivative comprising an insulin molecule and a reactive group for covalently bonding a blood component.

In a preferred embodiment of the present invention, the insulin molecule is of formula I:

FORMULA I

A Chain

A1

H2N—Gly Ile Val Glu Gln Cys Cys Thr Ser Ile Cys Ser Leu Tyr Gln Leu Glu Asn Tyr Cys Asn—CO2H (SEQ ID NO: 1)

S
S

S
S

S
S

H2N—Phe Val Asn Gln His Leu Cys Gly Ser His Leu Val Glu Ala Leu Tyr Leu Val Cys Gly Glu Arg Gly Phe Phe Tyr Thr Pro Lys Thr

B1

B Chain

B29

CO2H
(SEQ ID NO: 2)

and the reactive group is coupled to an amino acid of the insulin molecule at a position selected from the positions Gly A1,Phe B1 and Lys B29.

In a preferred embodiment of the present invention, the reactive group selected are from the group consisting of Michael acceptors (α,β, unsaturated carbonyl moiety) succinimidyl-containing group and maleimido-containing groups, more preferably MPA (3-MaleimidoPropionic Acid).

In a preferred embodiment of the present invention, the reactive group is coupled to an amino acid of the insulin molecule via a linker, such as, but not limited to (2-amino) ethoxy acetic acid (AEA), ethylenediamine (EDA), amino ethoxy ethoxy succinimic acid (AEES), AEES-AEES, 2-[2-(2-amino)ethoxy)] ethoxy acetic acid (AEEA), AEEA-AEEA, -NH₂-(CH₂)ₙ-COOH where n is an integer between 1 and 20 and alkyl chain (C1-C10) motif saturated or unsaturated in which could be incorporated oxygen nitrogen or sulfur atoms, such as, but not limited to glycine, 3-aminopropionic acid (APA), 8-aminooctanoic acid (OA) and 4-aminobenzoic acid (APhA)and combination thereof.

In a preferred embodiment of the present invention, the blood component is a blood protein, more preferably is serum albumin.

In accordance with the present invention, there is provided an insulin conjugate comprising an insulin derivative of the present invention and a blood component, wherein the reactive group and the blood component are conjugated through a covalent bond formed between said reactive group and said blood component. This conjugate is formed *in vivo* or *ex vivo.*

In accordance with the present invention, there is provided a pharmaceutical composition comprising the insulin derivative of the present invention in association with a pharmaceutically acceptable carrier.

In accordance with the present invention, there is provided a pharmaceutical composition comprising the insulin conjugate of the present invention in association with a pharmaceutically acceptable carrier.

In accordance with the present invention, there is provided a method for treating a glycaemic-related disease or disorder in a subject suffering from said glycaemic-related disease or disorder, comprising administering at least one of the insulin derivatives of the present invention, the conjugate of the present invention and the pharmaceuticals compositions of the present-invention to the subject.

All references herein are hereby incorporated by reference.

### BRIEF DESCRIPTION OF THE DRAWINGS

Fig. 1 illustrates example I to example VIII derived from native human insulin;

Fig. 2 illustrates competitive binding of insulin, insulin derivatives and conjugate of insulin derivatives on liver membranes of wistar rats;

Fig. 3 illustrates competitive binding of insulin, insulin derivatives and conjugate of insulin derivatives on liver membranes of wistar rats;

Fig 4A, 4B, 4C 5A,5B 6A,6B present various phases of 3T3 L1 adipocytes differenciation stages

Fig. 7 illustrates glucose transport in 3T3-L1 adipocytes for insulin, example III and IV and their corresponding conjugate

Fig. 8 illustrates glucose transport in epididymal fat cells from wistar rats adipocytes for insulin, example III and VI and their corresponding conjugate

Fig. 9 illustrates delta glycaemia in function of time in animals treated with insulin at 3.6 mg/kg;

Fig. 10 illustrates delta glycaemia in function of time in animals treated with insulin derivatives of examples I, II and III at 3.6 mg/kg;

Fig. 11 illustrates delta glycaemia in function of time in animals treated with insulin derivatives of examples I, II and III at 17.9 mg/kg;

Fig. 12 illustrates delta glycaemia in function of time in animals treated with insulin and insulin derivative of example I at 3.6 mg/kg and insulin derivative of example I at 17.9 mg/kg;

Fig. 13 illustrates delta glycaemia in function of time in animals treated with insulin and insulin derivative of example II at 3.6 mg/kg and insulin derivative of example II at 17.9 mg/kg;

Fig. 14 illustrates delta glycaemia in function of time in animals treated with insulin and insulin derivative of example III at 3.6 mg/kg and insulin derivative of example III at 17.9 mg/kg;

Fig. 15 illustrates the pharmacokinetic profile of native insulin injected subcutaneously and intravenously;

Fig. 16 illustrates the pharmacokinetic profile of conjugate of example III injected subcutaneously and intravenously;

Fig. 17 illustrates the pharmacokinetic profile of example III injected subcutaneously and intravenously;

Fig. 18 illustrates comparative pharmacokinetic profile of insulin, example III and conjugate of example III injected subcutaneously;

Fig. 19 illustrates comparative pharmacokinetic profile of insulin, example III and conjugate of example III injected intravenously;

Fig. 20 illustrates comparative pharmacodynamic profile post subcutaneous injections of insulin, example I to IV and vehicule in streptozocin induced diabetic rat;

Fig. 21 illustrates comparative pharmacodynamic profile post subcutaneous injections of insulin, the conjugate of example I to IV and vehicule in streptozocin induced diabetic rat;

Fig. 22 illustrates comparative pharmacodynamic profile of repeated subcutaneous injections of example III (day1 versus day 6 versus dayl2 versus control); and

Fig. 23 illustrates comparative pharmacodymanic profile of repeated subcutaneous injections of the conjugate of example III (day1 versus day 6 versus day12 versus control).

### DETAILED DESCRIPTION OF THE INVENTION

In accordance with the present invention, there is provided a long tasting insulin derivative. More particularly, the insulin derivative comprises an insulin molecule and a reactive group coupled thereto, the reactive group being for covalently bonding a blood component. In the present application, it is intended that the covalently bonding, resulting in the formation of a conjugate insulin-reactive group -blood component can be formed *in vivo* upon administration of the insulin derivative of the present invention. It is also intended that the covalently bonding can occur *ex vivo* by contacting the insulin derivative of the present invention with a source of albumin that can be recombinant albumin, or extracted from a subject's plasma, or providing from any other suitable source, which source would be known by one skilled in the art.

The insulin molecule may be native human insulin (see the sequence of native human insulin below in Formula I) or an analogue thereof such as an insulin molecule with amino acid substitution(s), amino acid deletion(s) or amino acid addition(s). The following are listed as examples of insulin analogue that can be used in accordance with the present invention without the intention to limit the present analogue in any way: insulin glargine called Lantus® of Aventis Pharmaceuticals Inc., which has a glycine substituted in position A21 and two residues of arginine added in C-terminus of the chain B; insulin detemir called Levemir® of Novo Nordisk A/S, which is a native human insulin where threonine in position B30 is deleted and tetradecanoyl is added on the lateral chain of lysine B29; insulin lispro called Humalog® of Eli Lilly, which is Lys B28, Pro B29 human insulin; insulin aspart called NovoLog® of Novo Nordisk A/S, which Asp B28 human insulin; and insulin glulisine called Apidra® of Aventis, which is Lys B3, Glu B29 human insulin.

Leu Tyr Gln FORMULA I
Leu
Ser
Glu
A Chain
Cys
Ile Asn
S
S
A1 Ser Tyr
Thr
H2N— Gly Ile Val Glu Gln Cys Cys Cys Asn —CO2H (SEQ ID NO: 1)
S S
S S
H2N— Phe Val Asn Gln His Leu Cys Cys Gly Glu Arg Gly Phe
B1 Gly Val Phe
B Chain Ser Leu Tyr
His Tyr Thr
Leu Leu Pro
Val Glu Ala Lys B29
Thr
CO2H
(SEQ ID NO: 2)

The reactive group may be coupled to different functionalities on the insulin molecule or analogue thereof. Preferably, the reactive group is coupled to an available amino group of the insulin molecule, such as the α-amino groups of the N-terminus amino acid of chains A and B, or the ε-amino group of Lys B29. In accordance with the invention, insulin analogue containing substituted and/or added amino acid(s) may contain additional amino group for coupling the reactive group; or other functionalities appropriate for coupling the reactive group thereto. Preferred reactive groups capable to covalently bond a blood component in vivo or ex vivo, are Michael acceptors (*α,β,* unsaturated carbonyl moiety) succinimidyl-containing groups and maleimido-containing groups. The more preferred reactive group is a maleimido-containing group, and more particularly MPA (3-MaleimidoPropionic Acid).

Optionally, the reactive group is optionally coupled to the insulin molecule via a linker. The linker is preferably selected from the group consisting of hydroxyethyl motifs such as (2-amino) ethoxy acetic acid (AEA), ethylenediamine (EDA), amino ethoxy ethoxy succinimic acid (AEES), 2-[2-(2-amino)ethoxy)] ethoxy acetic acid (AEEA), AEEA-AEEA, - NH2-(CH2)n-COOH where n is an integer from 1 to 20; one or more alkyl chains (C1-C10) saturated or unsaturated in which could be incorporated oxygen nitrogen or sulfur atoms motifs such as glycine, 3-aminopropionic acid (APA), 8-aminooctanoic acid (OA), 4-aminobenzoic acid (APhA). Examples of combinations of linkers include, without limitations, AEEA-EDA, AEEA-AEEA, AEA-AEEA, AEES-AEES, and the like. The preferred linker is 8-aminooctanoic acid (AOA) or the use of no linker with the reactive group MPA. One skilled in the art would readily know what type of linker is suitable for the purpose of the present invention.

The present invention also relates to an insulin conjugate. The conjugate comprises an insulin derivative where its reactive group has reacted with a blood component *in vivo* or *ex vivo* so as to form a covalent bond. Therefore, the conjugate may be formed *in vivo* by the administration of the insulin derivative, or *ex vivo* by contacting the insulin derivative to a blood solution or purified blood components *ex vivo* in conditions that allow formation of the covalent bond. Purified blood components can be provided by extraction and purification from blood sample or produced by recombinant techniques. The preferred blood component is a blood protein, and more preferably, serum albumin.

The present invention further relates to method for treating glycaemic-related diseases or disorders, comprising the administration of insulin derivatives or insulin conjugates. Glycaemic-related diseases or disorders include diabetes of Type I and II, and gestational diabetes. Also, cystic fibrosis, polycystic ovary syndrome, pancreatitis and other pancreas-related diseases may also be treated by the administration of insulin derivatives or insulin conjugates of the present invention. Insulin is also known as a growth factor and therefore, the insulin derivatives or insulin conjugates of the present invention can be useful in topical administration for wound healing and other related indications.

The following examples are for the purpose of further illustrating the invention as described above rather than for the purpose of limiting the scope of the present invention.

### EXAMPLES

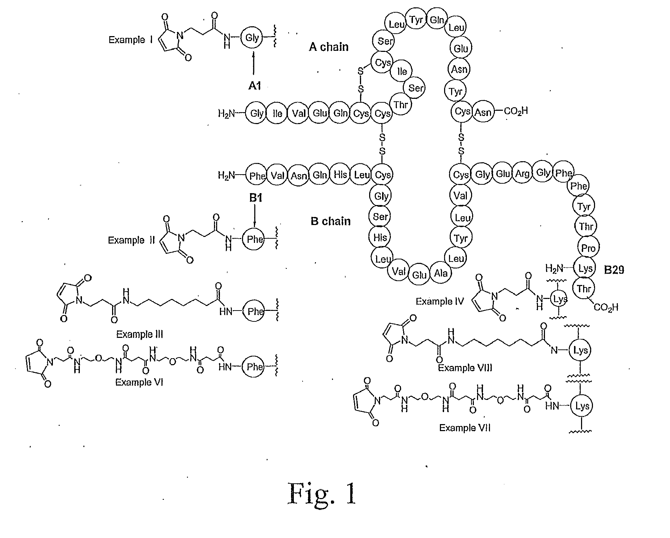

Fig. 1 illustrates the insulin molecule and the Gly A1, Phe B1 and Lys B29 sites referred to along the following examples.

### Example I

### Synthesis of (Gly A1)-MPA-Insulin

Insulin (100 mg) was dissolved in DMF(dimethylformamide) (2 mL) and TFA (100 uL). To the solution, NMM (4-methylmorpholine, 200 uL) and MPA-OSu (N-succinimidyl 3-maleimidopropanoate, 9.2 mg 2.5 equivalents) were added and the reaction was stirred for 2 h. The reaction was quenched by addition of water and adjusted to pH 4 with AcOH(acetic acid). Acetonitrile was added to dissolve the precipitate and the total volume of water/acetonitrile (3:1) was 20 mL. The solution was injected into a semi-preparative HPLC. Phenomenex Luna 10 m phenyl-hexyl 21 mm X 250 mm column equilibrated with an aqueous TFA solution (0.1% TFA in H₂O solvent A) and an acetonitrile TFA solution (0.1% TFA in acetonitrile, solvent B). Elution was achieved at 9.5 mL/min by running a 27 to 31% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions at Rt= 36-46 min. were collected, combined and lyophilized to give a white powder (40 mg) along with 23 mg of recovered insulin.

Mass calculated is 5958.5 g/mol, and measured by LC-MS is 5958.0 g/mol.

Table 1 show the amino acid sequence analysis (Edman degradation using phenylisothiocyanate) that was performed to confirm that the A-chain N-terminal was blocked and B-chain N-terminal (phenylalanine) was still free.

**Table 1 presents the structural elucidation via Edman degradation of example I to IV**

| **Table 1** | | | | | |
|---|---|---|---|---|---|
| **Compound** | **Chain** | **Edman Degradation Results (Positions)** | | | |
| | | **1** | **2** | **3** | **4** |
| Human Insulin | **A** | Gly | Ile | Val | Glu |
| | **B** | Phe | Val | Asn | Gln |
| Example I | **A** | - | - | - | - |
| | **B** | Phe | Val | Asn | Gln |
| Example II | **A** | Gly | Ile | Val | Glu |
| | **B** | - | - | - | - |
| Example III | **A** | Gly | Ile | Val | Glu |
| | **B** | - | - | - | - |
| Example IV | **A** | Gly | Ile | Val | Glu |
| | **B** | Phe | Val | Asn | Gln |

### Example II

### Synthesis of (Phe B1)-MPA-Insulin

Insulin (100 mg) was dissolved in DMSO (dimethylsulphoxide) (4 mL) and Et₃N (triethylamine) (100 uL) with sonication. To the solution, Boc₂O (Di-tert-butyl dicarbonate) (9.3 mg, 2.5 equivalents) was added and the reaction was stirred at ambient temperature for 30 min. The reaction was quenched by addition of water (15 mL) and acetonitrile (5 mL) and the solution was adjusted to pH 4 with AcOH. The solution was injected into a semi-preparative HPLC. Phenomenex Luna 10 m phenyl-hexyl 21 mm X 250 mm column equilibrated with an aqueous TFA solution (0.1% TFA in H2O, solvent A) and an acetonitrile TFA solution (0.1% TFA in acetonitrile, solvent B). Elution was achieved at 9.5 mL/min by running a 27 to 40% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. Three products (Boc-insulin, Gly A1 Lys B29-BisBoc-insulin and TrisBoc-insulin) were isolated and the fractions of desired (GlyA1 Lys B29)-BisBoc-insulin were combined and lyophilized to give a white powder (72 mg).

(Gly A1 Lys B29)-BisBoc-Insulin (51 mg) in DMF (3 mL) was reacted with MPA-OSu (36 mg) in the presence of Et₃N (30 uL). The reaction was stirred for 2 h at ambient temperature. DMF was evaporated by a under vacuum. The residue was treated with TFA (2 mL) for 10 min. and then TFA evaporated. The crude product was dissolved in water/acetonitrile (3:1) and the solution injected into a semi-preparative HPLC. Phenomenex Luna 10 m phenyl-hexyl 21 mm X 250 mm column equilibrated with an aqueous TFA solution (0.1% TFA in H2O, solvent A) and an acetonitrile TFA solution (0.1% TFA in CH3CN, solvent B). Elution was achieved at 9.5 mL/min by running a 27 to 32% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions were combined and lyophilized to give a white powder (29 mg).

Mass calculated is 5958.5 g/mol, and measured by LC-MS is 5958.4 g/mol.

The amino acid sequence analysis (Edman degradation using phenylisothiocyanate) was used to confirm that the B-chain N-terminal was blocked and A-chain N-terminal (glycine) was still free(see table 1).

### Example III

### Synthesis of (B1)-MPA-OA-insulin

(Gly A1 Lys B29)-BisBoc-insulin (39 mg) in DMF (3 mL) and Et₃N (30 uL) was reacted with MPA-OA-OSu ([N-succinimidyl 8-N-(3-maleimidopropanylcarbonyl)AminoOctanoate] 25 mg ) for 4 h. DMF was evaporated and the residue treated with TFA for 10 min. After evaporation of TFA, the residue was dissolved in water/acetonitrile (1:3). The solution was injected to a semi-preparative HPLC. Phenomenex Luna 10 m phenyl-hexyl 21 mm X 250 mm column equilibrated with an aqueous TFA solution (0.1% TFA in H2O, solvent A) and an acetonitrile TFA solution (0.1% TFA in acetonitrile, solvent B). Elution was achieved at 9.5 mL/min by running a 27 to 36% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions were combined and lyophilized to give a white powder (21 mg).

Mass calculated is 6099.5 g/mol, and measured by LC-MS is 6099.6 g/mol.

### Example IV

### Synthesis of (Lys B29)-MPA-Insulin

Insulin (74 mg) was dissolved in DMSO (2 mL) and AcOH (46 uL). To the solution Boc₂O (6.9 mg, 2,5 equivalents) was added and the reaction was stirred for 5 h at room temperature. Water (15 mL) and acetonitrile (5 mL) were added and the solution was injected to a semi-preparative HPLC column (C18 phenyl-hexyl) in flow rate of 9.5 mL/min and with gradient from 27-40% over 120 min. The fractions at 43 min. were combined and lyophilized to give (Gly A1 Phe B1)-Boc2-Insulin (30 mg).

(Gly A1 Phe B1)-Boc₂-Insulin (30 mg) in DMF (2 mL) and NMM (4-methylmorpnoline, 100 uL) was reacted with MPA-OSu (10 mg) for 60 min. DMF was evaporated and the residue treated with TFA for 10 min. The residue was dissolved in water/acetonitrile (3:1) and the solution injected to a semi-preparative HPLC. Phenomenex Luna 10 m phenyl-hexyl 21 mm X 250 mm column equilibrated with an aqueous TFA solution (0.1% TFA in H₂O solvent A) and an acetonitrile TFA solution (0.1% TFA in acetonitrile, solvent B). Elution was achieved at 9.5 mL/min by running a 27 to 32% B gradient over 120 min. Fractions containing peptides were detected by UV absorbance at 214 and 254 nm. Fractions were collected in 9.5 mL aliquots. Fractions containing the desired product profile were identified by mass detection after direct injection onto LC/MS. The pure fractions were combined and lyophilized to give a white powder (22.2 mg).

Mass calculated is 5958.5 g/mol, and measured by LC-MS is 5958.0 g/mol.

The amino acid sequence analysis (Edman degradation using phenylisothiocyanate) was used to confirm that both B-chain (phenylalanine) and A-chain (glycine) N-termini were free (see table 1).

### Example V

### Synthesis of MPA-(AEES)₂-COOH linker

Flash column chromatography was carried out using a Biotage® "40i flash chromatography" modular system. Semi preparative HPLC purifications were done on a Waters "Breeze" system 1500 series using a Phenomex luna (RP-18, 10 u phenyl-hexyl 250 X 21.2 mm) column with a 9.5 mL/min mobile phase flow rate. A Gilson 690 system was used for preparative scale purification using a Phenomex luna (RP-18, 10 u phenyl-hexyl 250 X 50.0 mm) column with a 50 mL/min mobile phase flow rate. A Gradient of acetonitrile (CH₃CN) (0.1 %TFA) in water (0.1 %TFA) was used with further details indicated in each compound's synthetic procedure. LC-MS was performed using an Agilent 1100 series LC-MSD single quadrupole mass spectrometer with an ES1 electrospray source.

### MPA-(AEES)₂-COOH linker synthesis

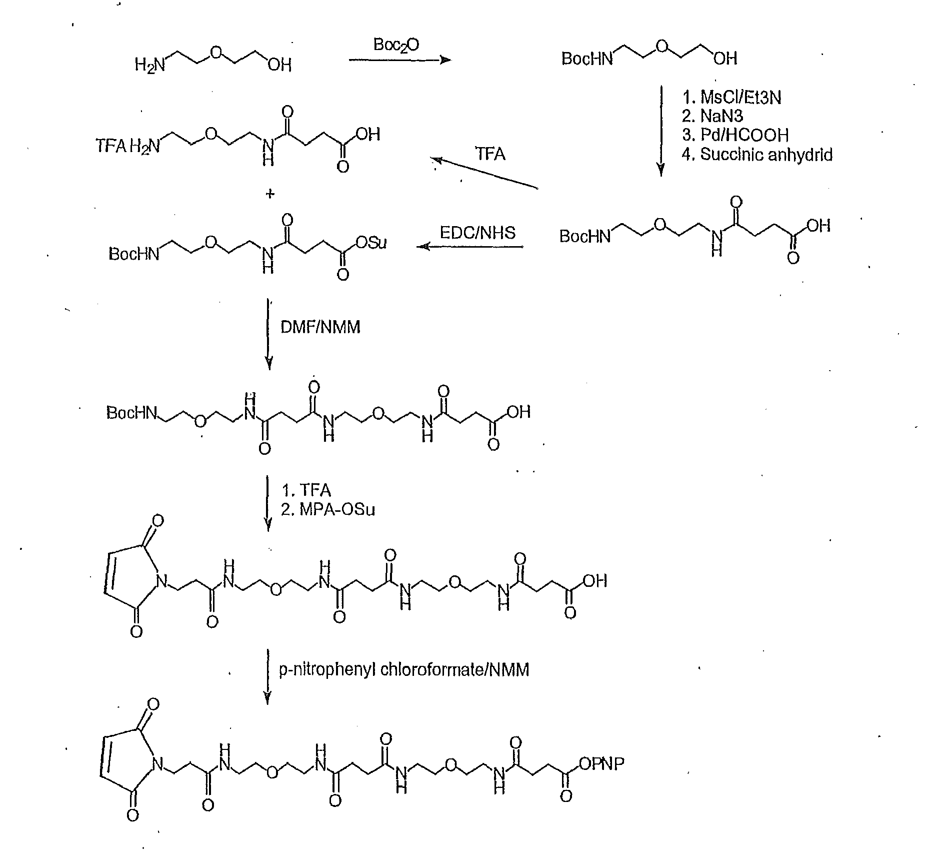

### Example VI

### Synthesis of (Phe B1)-MPA-(AEES)₂-Insulin

2-(2-Aminoethoxy)ethanol (50.0 g) in methanol (150 mL) was reacted with Boc₂O (93.4 g) for 30 min. The methanol was evaporated in vacuo and the residue was taken up in ethyl acetate, washed with water, brine and dried with sodium sulfate. After evaporation of the solvent, the crude product was used for the next step. MS m/z 205.

The protected alcohol was dissolved in N,N-dimethyl formamide (500 mL) in the presence of Et₃N (66 mL). MsCl (Mesylate chloride) (33.4 mL) was added dropwise at 0°C over 30 min and then stirred at ambient temperature for 1 h. NaN₃ (127.6 g) was then added to the reaction mixture followed by NMM (N-methylmorpholine, 215 mL) and the reaction was stirred at 40-50°C for 16 h. The reaction mixture was poured into ethyl acetate (2L) and washed with water. The water layer was back extracted with ethyl acetate (2L) and the combined ethyl acetate layers were washed with water, brine and dried. After evaporation of the solvent, the crude product was used for the next step (101.4 g contains some DMF). MS m/z 231.

The crude product (62.4 g) was dissolved in methanol (300 mL) followed by the addition of Pd(OAc)₂ (3.0g) and formic acid (96% 62 g). After completion of the reaction, Pd species were removed by filtration through celite. The methanol was removed in vacuo and dried further under vacuum. The crude product was used in the next step.

The crude product was dissolved in dichloromethane and neutralized by Et₃N until it is basic. Succinic anhydride (32.3 g) was added in one portion. The reaction was stirred at ambient temperature for 1 h. The solvent was removed in vacuo and the residue acidified by HCl 1 N to pH 3. The product was extracted with ethyl acetate. The ethyl acetate layer was passed through a silica gel plug (1 kg). Then the silica gel was washed with 2-4% methanol in ethyl acetate. The pure fractions (as judged by thin layer chromatography) were combined and the solvent removed in vacuo to give Boc-AEES as an oily residue (36 g, 44%). MS m/z 305

Boc-AEES (5.5 g) was treated with N-hydroxysuccinimide (NHS, 4.57 g) and ethyl-(dimethylaminopropyl)carbodiimide hydrochloride (EDC, 7.62 g) in dichloromethane (30 mL) for 2 h. The NHS ester was poured into ethyl acetate (500 mL), washed with 0.1 N HCl and dried with sodium sulfate. The solvent was removed in vacuo and the residue used as such in the next step.

Boc-AEES (6.05 g) was treated with trifluoroacetic acid (TFA, 10 mL) for 10 min. TFA was removed in vacuo and dried further under vacuum. The (AEES) amino acid in was dissolved in N,N-dimethylformamide (20 mL) and basified with excess NMM. The crude product from example V was then added and the reaction was stirred at ambient temperature for 1 h. The solvent was evaporated under reduced pressure and the residue was injected into preparative. HPLC using a 5-40% gradient over 60 min. The solvent was removed and the residue dried under vacuum to give Boc-(AEES)₂-COOH as an oil (5,64 g, 84%). MS m/z 478.

Boc-(AEES)₂-COOH (3.60 g) was treated with trifluoroacetic acid (TFA, 10 mL) for 10 min. TFA was removed in vacuo and dried further under vacuum. The (AEES)₂ amino acid in was dissolved in N,N-dimethylformamide and basified with NMM. MPA-OSu (2.94 g) was added and the mixture was stirred for 30 min. N,N-dimethylformamide was removed under vacuum. The residue was dissolved in water and injected into preparative HPLC using a 5-40% gradient over 60 min. The pure fraction were combined and the solvent was removed to give MPA-(AEES)₂-COOH as an off-white solid (3.8 g, 95%). MS m/z 542.2.

MPA-(AEES)₂-COOH (3.04 g) was dissolved in N,N-dimethylformamide (20 mL) and the presence of NMM (1.13 mL) and treated with p-nitrophenyl chloroformate (1.13 g). The reaction mixture was stirred at ambient temperature for 2 h. N,N-dimethylformamide was removed under vacuum. The residue was purified by flash column chromatography using Biotage® system. The column was rinsed with ethyl acetate (500 mL) followed by 10% methanol in ethyl acetate (1 L). The pure fractions were combined and the solvent removed to give MPA-(AEES)₂-CO₂PNP as a solid (1.39 g, 37%). MS m/z 663.

Gly A1, Lys B29-BisBoc-Insulin (200 mg) in N,N-dimethylformamide (10 mL) was coupled with MPA-(AEES)₂-CO₂PNP from example VIII (200 mg) in the presence of NMM (200 uL) at ambient temperature for 16 h. N,N-dimethylformamide was removed under vacuum and the residue treated with TFA for 10 min. TFA was removed in vacuo, the residue was dissolved in water and injected into HPLC using a 27-32% gradient over 120 min. The pure fractions were combined and the solvent lyophilized to give ( Phe B1)-MPA(AEES)₂-Insulin as a white powder (95.0 mg, 43.7%). MS m/z 6330.4.

### Example VII

### Synthesis of (B29)-MPA(AEES)2-Insulin

Gly A1 Phe B1-BisBoc-Insulin (200 mg) in N,N-dimethylformamide (10 mL) was coupled with MPA-(AEES)₂-CO2PNP as from Example VI (200 mg) in the presence of NMM (200 uL) at ambient temperature for 16 h. N,N-dimethylformamide was removed under vacuum and the residue treated with TFA for 10 min. After TFA was removed in vacuo, the residue was dissolved in in water and injected into HPLC using a 27-32% gradient over 120 min. The pure fractions were combined and the solvent lyophilized to give ( Lys B29)-MPA(AEES)₂-Insulin as a white powder (56.3 mg, 25.9%). MS m/z 6328.8.

### Example VIII

### Synthesis of (B29)-MPA(OA)-insulin

Gly A1 Phe B1-BisBoc-Insulin (205 mg) in N,N-dimethylformamide (10 mL) was coupled with MPA-OA-CO₂Su (139 mg) in the presence of NMM (20 uL) at ambient temperature for 16 h. The solvent was removed under vacuum and the residue treated with TFA for 10 min. After TFA was removed in vacuo, the residue was dissolved in in water and injected into HPLC using a 27-36% gradient over 120 min. The pure fractions were combined and the solvent lyophilized to give ( Lys B29)-MPA(OA)-Insulin as a white powder (64.2 mg, 31%). MS m/z 6098.8.

### Example IX

### In vitro binding assays

Liver membranes of Wistar rats were incubated with [125I] insulin and increasing concentrations of insulin, DAC:insulin as described in Fig. 1 and their corresponding conjugate for 16 hours at 4°C. The membranes were filtered and washed 3 times and the filters were counted to determine [125I] insulin specifically bound. IC50 were calculated using GraphPad Prism software.

The results of IC50 are illustrated in Table 2.

**Table 2**

| | IC50 (nM) |
|---|---|
| Insulin | 12.0 |
| Example I | 57.1 |
| Example II | 18.8 |
| Example III | 17.2 |
| Example IV | 46.0 |
| Example VI | 24.9 |
| Example VII | 58.1 |
| Example VIII | 64.1 |
| Conjugate of Example I | 2059.0 |
| Conjugate of Example II | 100.5 |
| Conjugate of Example III | 87.7 |
| Conjugate of Example IV | 1190.0 |
| Conjugate of Example VI | 38.5 |
| Conjugate of Example VII | 508.2 |
| Conjugate of Example VIII | 1013.4 |

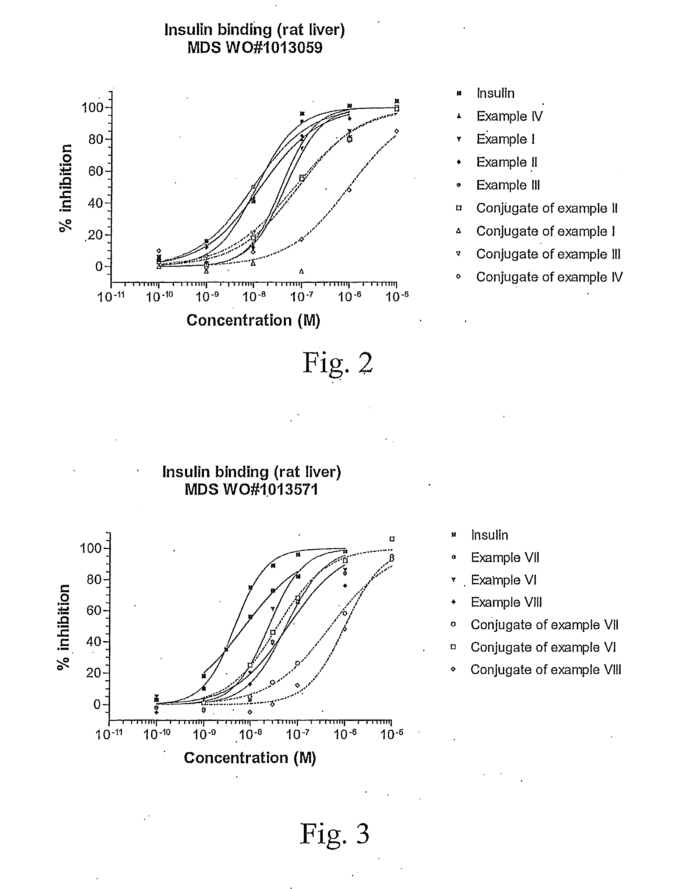

Fig. 2 and Fig. 3 illustrates the insulin binding in rat liver membrane in terms of the % of inhibition in function of the concentration of the insulin derivative of the present invention.

### Example X

### In vitro bioactivity

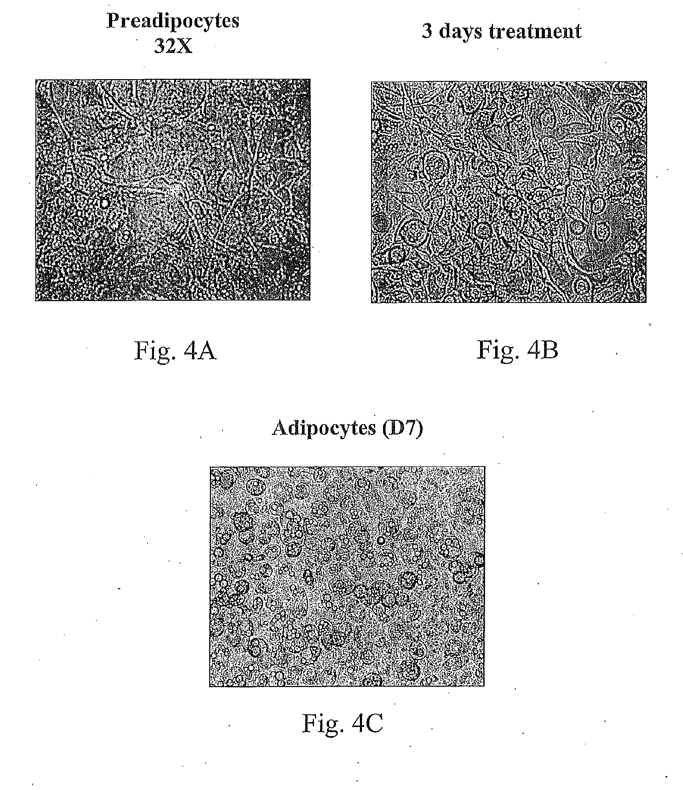

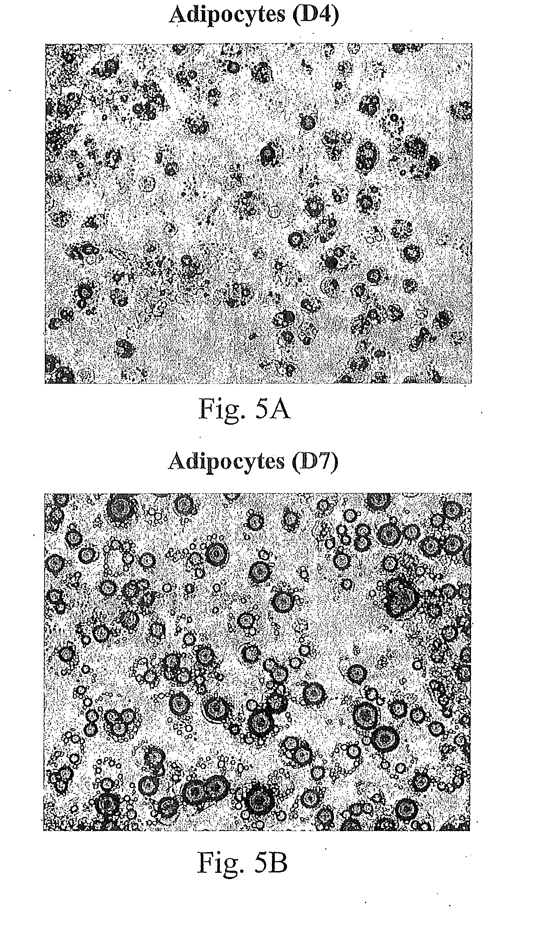

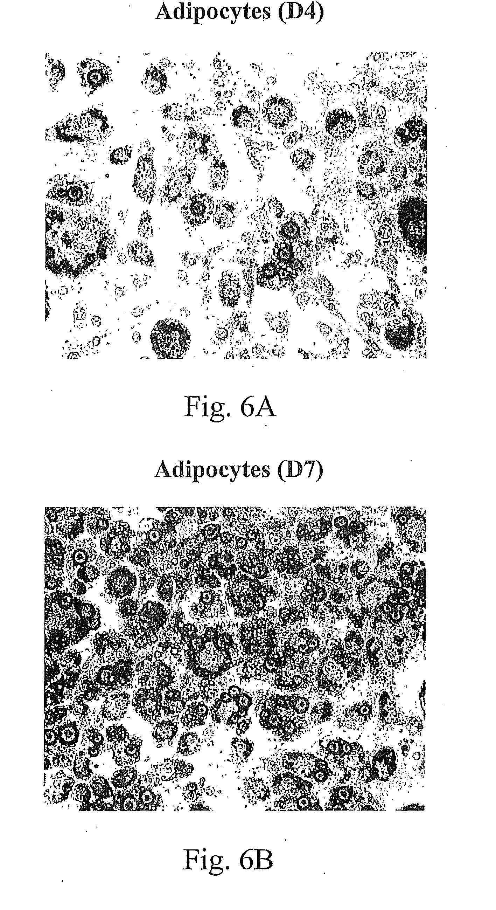

Glucose uptake in adipocytes was used to evaluate the in vitro activity. 3T3-L1 cells, a murine fibroblast cell line was differenciated in adipocytes for used in the bioassay. 3T3-L1 cells were plated and grown to confluency in DMEM and 10% FBS, followed by an incubation for two days. Differentiation was induced by adding dexamethasone and insulin (D0). By day 7, more than 90% of the cells displayed an adipocyte phenotype, i.e. accumulation of lipid droplets. Figs. 4A-4C show preadipocytes, cells after 3 days and adipocytes at 7 days. Figs. 5A and 5B show oil red o staining of adipocytes at 4 days and adipocytes at 7 days. Figs. 6A and 6B show oil red o and methylene blue staining at 4 days and at 7 days.

3T3-L1 adipocytes were starved overnight in DMEM containing 5mM glucose and 0.5% FBS. Cells were rinsed in Kreb's-Ringer-Hepes buffer containing 1% BSA and incubated with increasing concentrations of insulin, DAC:insulin derivatives and their corresponding conjugate for 20 minutes at 37°C and witch [¹⁴C]-2-deoxy-D-glucose (1µCi/well) for an additional 20 minutes. Cells were solubilized and radioactivity was measured. Glucose uptake (%) was calculated versus insulin control and EC50 were calculated using GraphPad PrisM™ software.

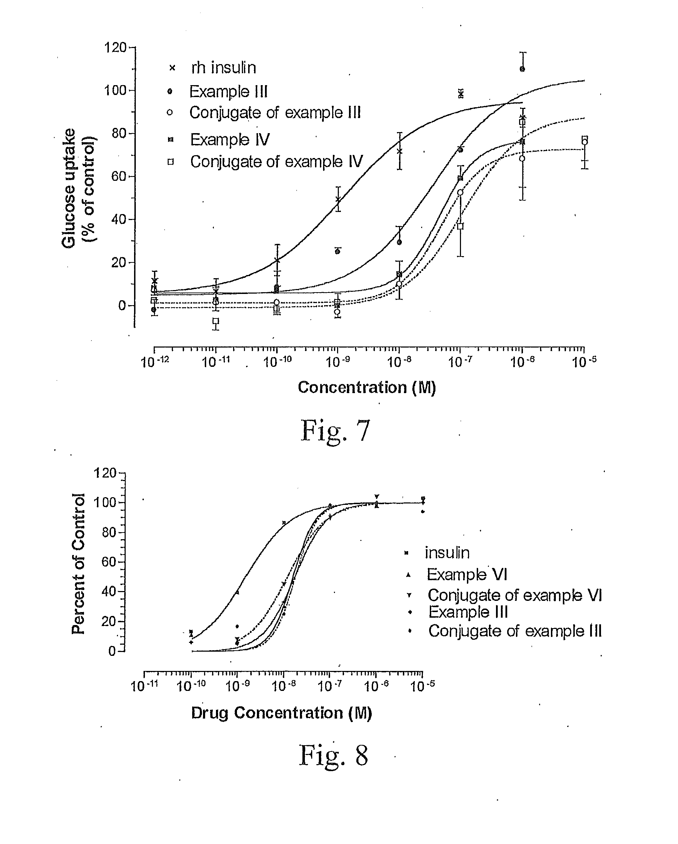

Table 3 show the EC50 results for the compounds tested and Fig. 7 illustrates the glucose uptake in % of control in function of the concentration (M) of the compounds.

**Table 3**

| **Compound** | **EC50 (nM)** |
|---|---|
| Human insulin | 1.2 |
| Example III Conjugate of Example III | 34 48 |
| Example IV Conjugate of Example IV | 45 110 |

### Example XI

In addition, glucose uptake in an other source of adipocytes was used to evaluate the in vitro activity. Epididymal fat obtained from Wistar derived male rats weighing 175 ± 25 g is used. The tissue (0.03 g/ml) is degraded by collagenase in modified HEPES solution pH 7.4 at 37°C. Test compound and/or vehicle is incubated with 500 µl aliquots in modified HEPES buffer pH 7.4 and D-[3-3H]Glucose (2.5 µCi/ml) is then added for 2 hour incubation. Test compound-induced the increase of glucose incorporation by more than 50 percent or more (≥50%) relative to the control 2 nM insulin response, indicates possible insulin receptor agonist activity. Test compound inhibition of the insulin-induced glucose incorporation response by more than 50% indicates insulin receptor antagonist activity. Compounds are screened at 10, 1, 0.1, 0.01 and 0.001 µM.

Table 4 show the EC50 results for the compounds tested and Fig. 8 illustrates the glucose uptake in % of control in function of the concentration (M) of the compounds.

**Table 4**

| **Compound** | **EC50 (nM)** |
|---|---|
| Human insulin | 1.4 |
| Example III Conjugate of Example III | 17.5 17.8 |
| Example VI Conjugate of Example VI | 15.4 13.3 |

### Example XII

### In vivo experiments

Evaluation the blood glucose lowering efficacy of recombinant human insulin versus and insulin derivatives of the present invention when administered subcutaneously to diabetic female db/db mice, are compared.

Tested compounds were administered by a single subcutaneous bolus injection in 5-6 week-old female db/db mice weighing 24.3 to 33.3 g. The average volume of dosing solution injected was 0.35 mL/mouse (12.5 mL/kg).

Recombinant (E. coli) human insulin (called herein below "rH insulin") is provided by ICNTM at a concentration of 28 IU/mg.

Stock solutions of insulin derivatives were prepared at 14.29 mg/mL (- 400 IU/mL) by reconstituting the synthesized insulin derivatives with acidified water (- pH 2). Stock solutions were subsequently diluted with 0.9% NaCl and 0.22 µm filtered (Millex GV) to obtain the dosing solutions shown in Table 5. Group 1, received 0.9% NaCl USP as control solution.

**Table 5**

| **Groups** | **Tested Compounds** | **Actual Solution Concentration (mg/mL)** |
|---|---|---|
| 2 | rH Insulin | 0.29 |
| 3 | Example I | 0.29 |
| 4 | Example I | 1.43 |
| 5 | Example II | 0.29 |
| 6 | Example II | 1.43 |
| 7 | Example III | 0.29 |
| 8 | Example III | 1.43 |

Groups and treatments are summarized in the Table 6.

**Table 6**

| **Groups** | **Test/Control Articles** | **Dose Level (mg/kg)** | **Dose Equivalence *(∼IU/kg)** | **Number of Animals (females)** |
|---|---|---|---|---|
| 1 | 0.9% NaCl | 0 | 0 | 5 |
| 2 | rH insulin | 3.6 | 100 | 5 |
| 3 | Example I | 3.6 | 100 | 5 |
| 4 | Example I | 17.9 | 500 | 5 |
| 5 | Example II | 3.6 | 100 | 5 |
| 6 | Example II | 17.9 | 500 | 5 |
| 7 | Example III | 3.6 | 100 | 5 |
| 8 | Example III | 17.9 | 500 | 5 |

| | | | | |
|---|---|---|---|---|
| * Based on the potency of the rH insulin estimated at 28 IU/mg by the vendor. | | | | |

Blood sampling (one drop) was performed via the tail tip and glucose levels were determined using a hand-held glucometer (Model: One Touch UltraTM, Lifescan Canada). Blood glucose levels were determined from all animals once prior to administration (pre-dose), and at 1, 2, 3, 4, 6, 24, 30, 48 and 72 hours post-dose.

### In vivo results:

All animals appeared normal prior to administration of the tested compounds. Approximately one hour post-dose, Group 2 of animals treated subcutaneously with 100 IU/kg of recombinant human insulin (rH insulin), exhibited slight decrease in activity and uncoordinated gait. Other treated animals appeared normal throughout the experiment. A slight decrease of food consumption was observed in animals treated with 17.9 mg /kg of compound of Example III.

Table 7 shows food consumption (total weight/cage (g)) following a single administration of rH insulin and insulin derivatives.

**Table 7**

| **Food consumption: total weight/cage (g)** | | | | | | |
|---|---|---|---|---|---|---|
| **Groups** | **Treatment** | **24h pre-dose** | **0-6h** | **0-24h** | **24-48h** | **48-72h** |
| 1 | 0.9% NaCl | 30.4 | 6.2 | 30.7 | 28.9 | 31.6 |
| 2 | rH insulin 3.6 mg/kg | 30.2 | 6.6 | 29.2 | 30.7 | 31.7 |
| 3 | Example I 3.6 mg/kg | 28.7 | 5.8 | 30.5 | 28.8 | 28.7 |
| 4 | Example I 17.9 mg/kg | 29.8 | 5.1 | 28.3 | 31.3 | 31.5 |
| 5 | Example II 3.6 mg/kg | 31.8 | 6.4 | 30.3 | 29.8 | 31.0 |
| 6 | Example II 17.9 mg/kg | 30.5 | 6.4 | 31.2 | 26.5 | 29.7 |
| 7 | Example III 3.6 mg/kg | 29.9 | 6.0 | 31.4 | 31.9 | 31.8 |
| 8 | Example III 17.9 mg/kg | 28.7 | 4.5 | 27.0 | 22.8 | 24.5 |

Table 8 shows food consumption versus control (total weight/cage (g)) following a single administration of rH insulin and insulin derivatives.

**Table 8**

| **% Food consumption *versus* control group** | | | | | | |
|---|---|---|---|---|---|---|
| **Groups** | **Treatment** | **24h pre-dose** | **0-6h** | **0-24h** | **24-48h** | **48-72h** |
| 1 | 0.9% NaCl | 100.0 | 100.0 | 100.0 | 100.0 | 100.0 |
| 2 | rH insulin 3.6 mg/kg | 99.3 | 106.5 | 95.1 | 106.2 | 100.3 |
| 3 | Example I 3.6 mg/kg | 94.4 | 93.5 | 99.3 | 99.7 | 90.8 |
| 4 | Example I 17.9 mg/kg | 98.0 | 82.3 | 92.2 | 108.3 | 99.7 |
| 5 | Example II 3.6 mg/kg | 104.6 | 103.2 | 98.7 | 103.1 | 98.1 |
| 6 | Example II 17.9 mg/kg | 100.3 | 103.2 | 101.6 | 91.7 | 94.0 |
| 7 | Example III 3.6 mg/kg | 98.4 | 96.8 | 102.3 | 110.4 | 100.6 |
| 8 | Example III 17.9 mg/kg | 94.4 | 72.6 | 87.9 | 78.9 | 77.5 |

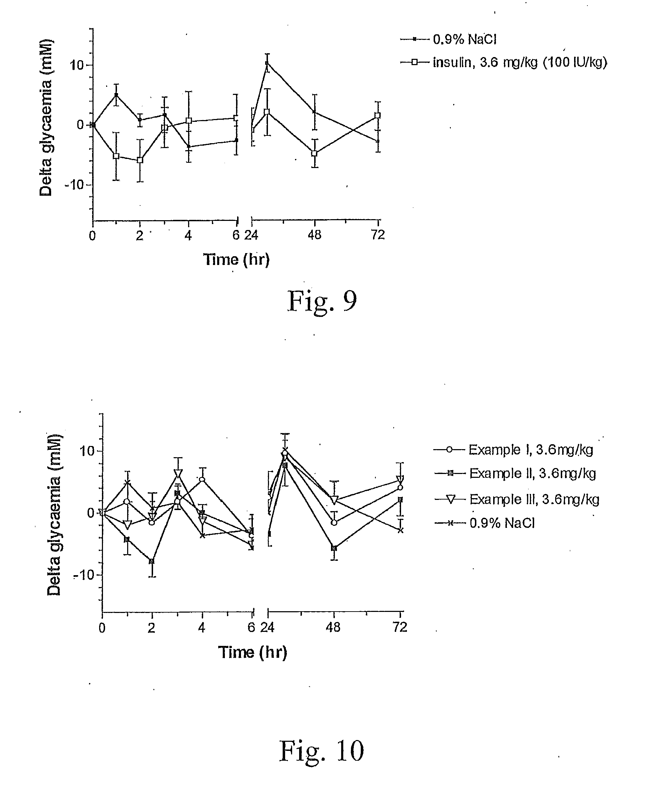

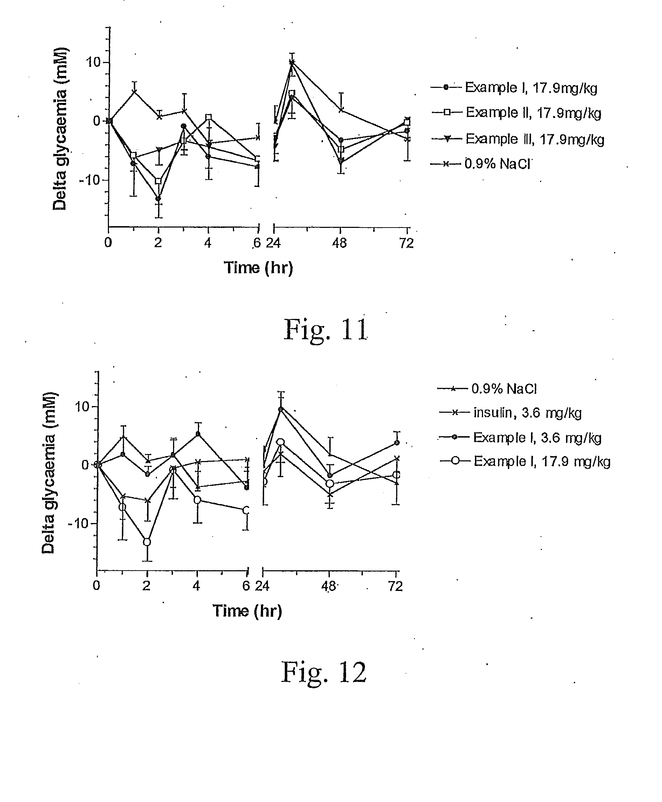

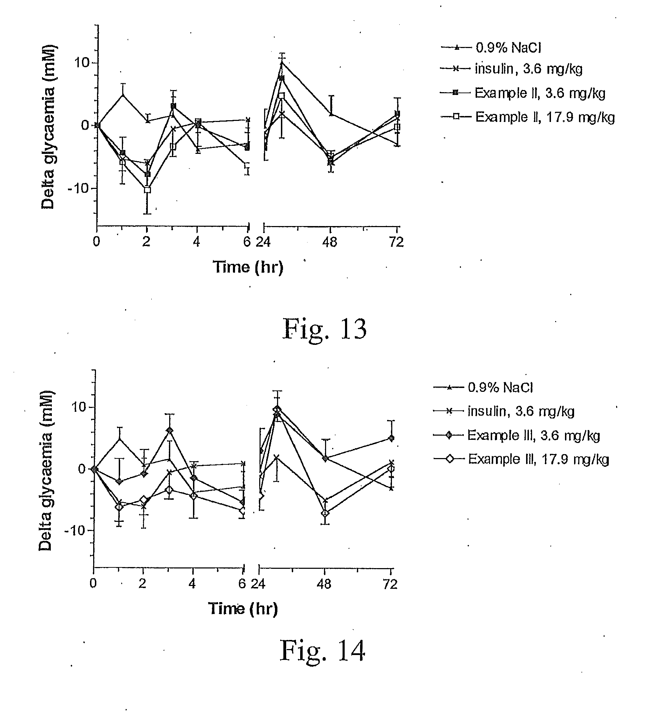

Delta glycaemia is calculated from blood glucose levels of post-dose glucose level versus the pre-dose glucose level for each individual mouse are reported in Figs 9, 10, 11, 12, 13, and 14. In general, insulin derivatives (Example I, Example II and Example III) were able to lower blood glucose concentrations in a dose-dependent manner and recombinant insulin, tested at one dose level only (100 IU/kg), was active for 2 hours. At 3.6 mg/kg, Example 2 was as active as insulin during the first 2 hours while only a marginal effect was observed with Example I and Example III. The lowering effect of rH insulin was more pronounced at 17.9 mg/kg since it was observed for up to 24 hours. Although the overall picture tends to demonstrate that insulin derivatives were active for up to 24 hours (as compared to the control group), it is important to note that glucose levels decreased at 1-2 hours post-dose then increased at 3-4 hours and decreased again at 6 hours. This 'up and down' response might indicate that the feeding habits and the metabolism of the mice are important parameters that can affect the efficacy of the drug.
It is important to mention that db/db mice develop insulin resistance with age, which could explain the very high dose of insulin that had to be injected to observe glucose lowering effect.

### Example XIII

### Pharmacokinetic profile in normal rats

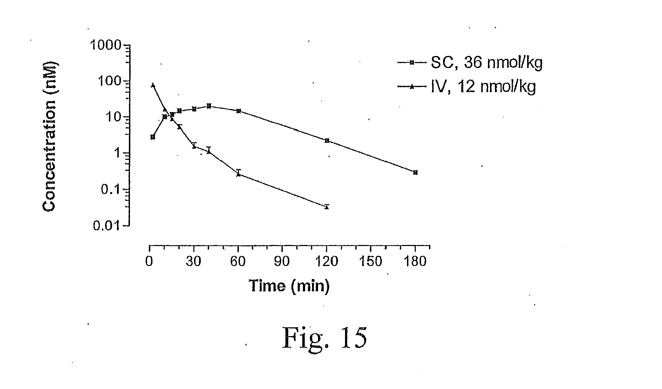

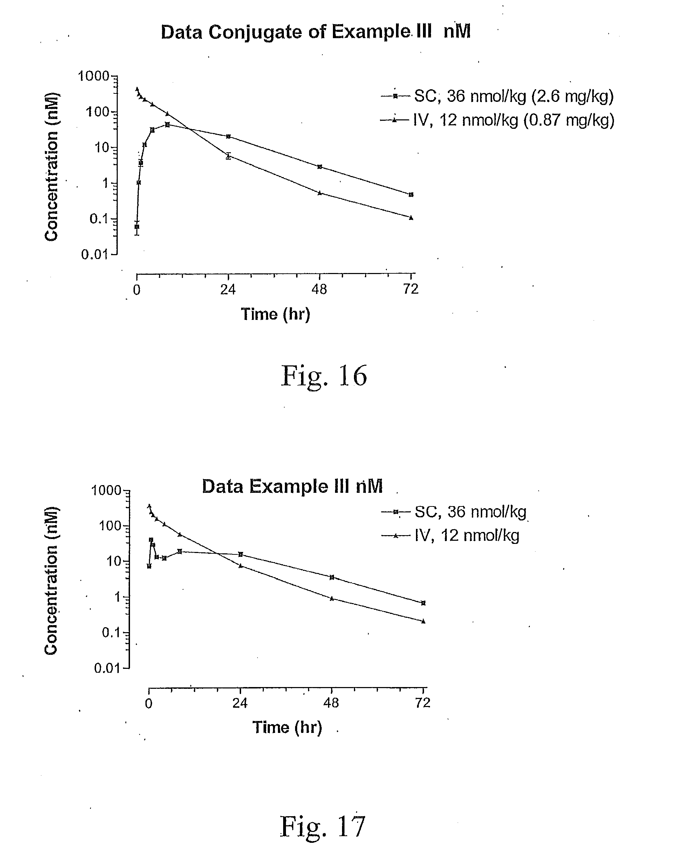

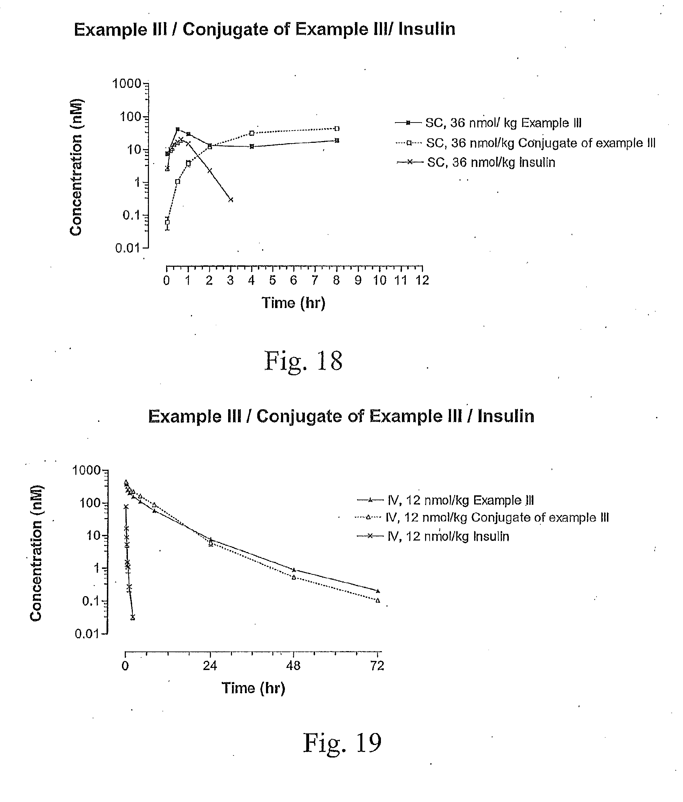

Rh insulin, the insulin derivative of example III and the conjugate of the insulin derivative of Example III were administered to 7-8 week-old male CD rats either at 36 nmol/kg sc or 12 nmol/kg intravenously (iv). Blood samples were collected up to 72 hours (only up to 3 hours for rh insulin-treated animals). The drug levels were determined using a human insulin ELISA kit (Linco). The pharmacokinetic parameters were calculated by non-compartmental analysis using the WinNonlin software, N=4 rats per compound/route. Fig. 15 is the pharmacokinetic profile of insulin in normal SD rats where insulin was administered sc at 36 nmol/kg and iv at 12 nmol/kg. Fig. 16 is the pharmacokinetic profile of the conjugate of example III in normal SD rats where the conjugate was administered sc at 36 nmol/kg and iv at 12 nmol/kg. Fig. 17 is the pharmacokinetic profile of the insulin derivative of example III in normal SD rats where the insulin derivative was administered sc at 36 nmol/kg and iv at 12 nmol/kg. Fig. 18 is the subcutaneous PK profile of the administration of insulin, insulin derivative of example III and the conjugate of the insulin derivative of example III. Fig. 19 is the intravenous PK profile of the administration of insulin, insulin derivative of example III and the conjugate of the insulin derivative of example III.

### Example XIV

### Single dose pharmacodynamic in diabetic rats

Diabetes was induced in male CD rats with a single i.v. injection of streptozotocin (60 mg/kg). Two days later, rats received a single sc injection of DAC™:Insulin derivatives at 120 nmol/kg, preformed conjugates at 300 nmol/kg, rh insulin at 20U/kg (120 nmol/kg) or vehicle. Blood glucose level were measured with a hand-held glucometer just prior injection and a 1, 2, 3, 4, 6, 8, 10, 11, 24, 30 and 48 hours postdose with 5 rats/groups except for vehicle were 3 rats/ group were tested. Glycemia of normal rats ranged from 5.2 to 7.6 mmol/L.

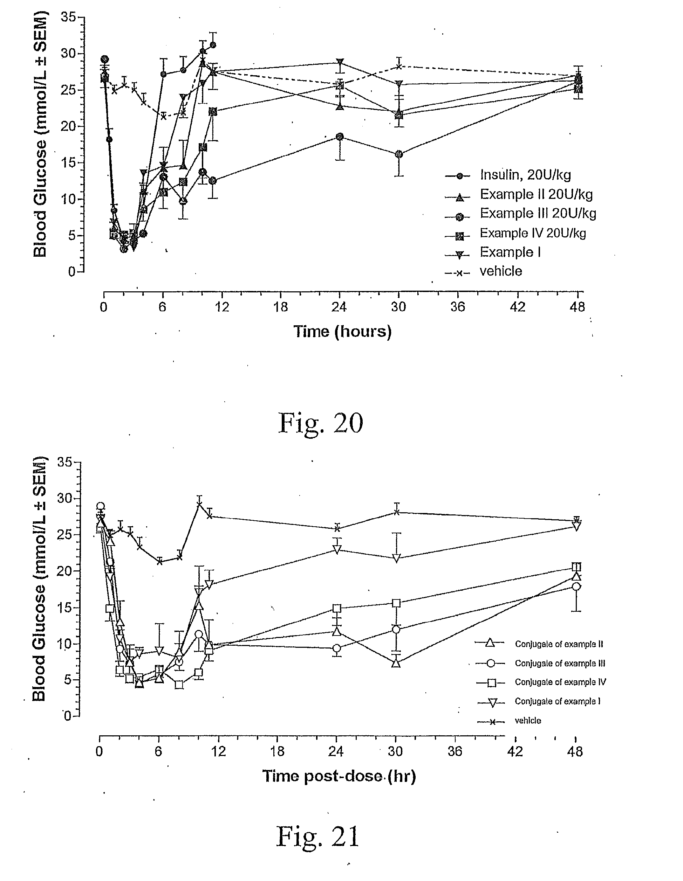

Figs. 20 shows the blood glucose level in rats after administration of 120nmol/kg of example I to IV. Fig 21 shows the blood glucose level in rats after administration of the corresponding conjugate of example I toIV of the present invention at 300nmol/kg.

### Example XV

### Evaluation of the potency of a DAC:Insulin derivative and its corresponding preformed conjugate following repeated subcutaneous administration

This assay was conducted to evaluate the potency of the insulin derivative of the example III and its conjugate versus free human recombinant insulin following repeated subcutaneous injections in adult male CD® rats.

Type 1 diabetes was induced in male CD® rats on study day 1 by a single intravenous (i.v.) injection of streptozotocin (60 mg/kg, pH 4.5). Hyperglycemia was confirmed using a blood glucose monitor to test blood. Test compound was administered once daily on study days 3 through 14 at 1 mL/kg body weight by subcutaneous (s.c.) injection. Blood glucose levels were tested just prior to dose administration each day and at 2,8 and 18 hours following administration. Feed and water consumption were monitored daily. Body weights were collected on study days 1, 3, 6, 9, 12, 15 and 17.

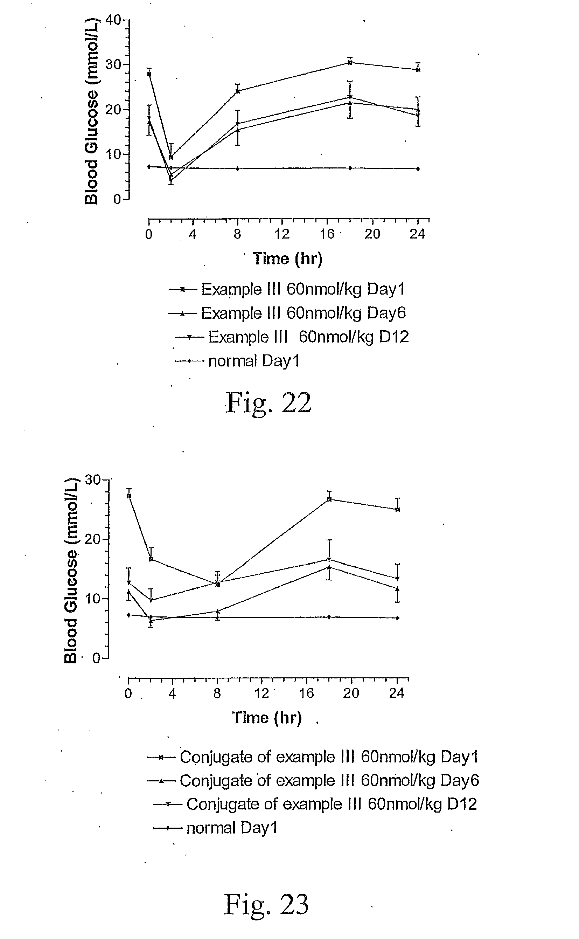

Fig. 22 illustrates the blood glucose daily profile at day 1, 6 and 12 for the insulin derivative of example III and Fig. 23 illustrates the blood glucose daily profile at day 1, 6 and 12 for the conjugate of the insulin derivative of example III.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

## Claims

1. An insulin derivative comprising an insulin molecule and a reactive group for covalently bonding a blood component.

2. The insulin derivative of claim 1, wherein the insulin molecule is of formula I: 
Leu Tyr Gln    FORMULA I
Ser    Leu
Glu
A Chain
Cys
S    Ile    Asn
A1
S    Ser    Tyr
Thr
H2N—Gly Ile Val Glu Gln Cys Cys    Cys Asn—CO2H
S    S
S    S
H2N—Phe Val Asn Gln His Leu Cys    Cys Gly Glu Arg Gly Phe
B1    Phe
Gly    Val
Ser    Leu    Tyr
B Chain
His    Tyr    Thr
Leu    Leu    Pro
Val Glu Ala    Lys    B29
Thr
CO2H
 and the reactive group is coupled to an amino acid of the insulin molecule at a position selected from the positions Gly A1, Phe B1 and Lys B29.

3. The insulin derivative of claim 1 or 2, wherein the reactive group is selected from the group consisting of Michael acceptor, a succinimidyl-containing group and a maleimido-containing group.

4. The insulin derivative of claim 3, wherein the Michael acceptor is an α,β unsaturated carbonyl moiety.

5. The insulin derivative of claim 1 or 2, wherein the reactive group is a maleimido-containing group.

6. The insulin derivative of claim 1 or 2, wherein the reactive group is 3-Maleimidopropionic acid (MPA).

7. The insulin derivative of any one of claims 1 to 6, wherein the reactive group is coupled to an amino acid of the insulin molecule via a linker.

8. The insulin derivative of claim 7, wherein said linker is selected from the group consisting of (2-amino) ethoxy acetic acid (AEA), ethylenediamine (EDA), amino ethoxy ethoxy succinimic acid (AEES), AEES-AEES, 2-[2-(2-amino)ethoxy)] ethoxy acetic acid (AEEA), AEEA-AEEA, -NH₂-(CH₂)ₙ-COOH where n' is an integer between 1 and 20 and alkyl chain (C1-C10) motif and combination thereof.

9. The insulin derivative of claim 8, wherein said alkyl chain (C1-C10) motif is one or more alkyl chains (C1-C10) saturated or unsaturated in which could be incorporated oxygen nitrogen or sulfur atoms.

10. The insulin derivative of claim 9, wherein said alkyl chain is selected from the group consisting of glycine, 3-aminopropionic acid (APA), 8-aminooctanoic acid (AOA) and 4-aminobenzoic acid (APhA).

11. The insulin derivative of claim 8, wherein said combination is selected from the group consisting of AEEA-EDA, AEEA-AEEA and AEA-AEEA.

12. The insulin derivative of claim 6, wherein said linker is -NH₂-(CH₂)₇-COOH.

13. The insulin derivative of claim 1 having the formula:

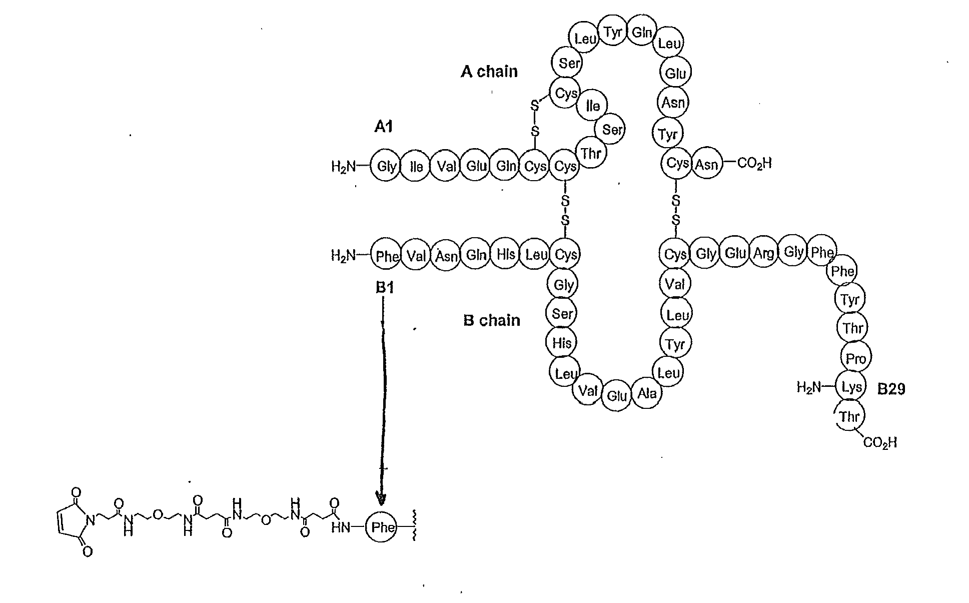
A chain
A1
H2N Gly Ile Val Glu Gln Cys Cys Ser Ile Cys Ser Leu Tyr Gln Leu Glu Asn Tyr Cys Asn CO2H
S
S
S
S
S
S
H2N Phe Val Asn Gln His Leu Cys Gly Ser His Leu Val Glu Ala Leu Tyr Leu Val Cys Gly Glu Arg Gly Phe Phe Tyr Thr Pro Lys Thr CO2H
B1
B chain
H2N
B29
O
O
O
O
O
O
O
O
N
H
O
N
H
N
H
O
N
H
HN Phe

14. The insulin derivative of claim 1, having the formula:

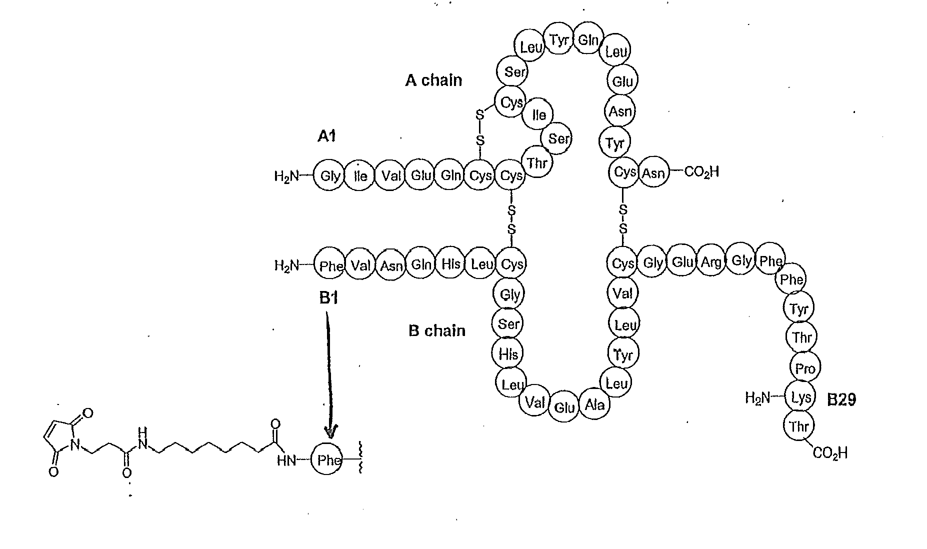
A chain
A1
H2N Gly Ile Val Glu Gln Cys Cys Thr Ser Ile Cys Ser Leu Tyr Gln Leu Glu Asn Tyr Cys Asn CO2H
S
S
S
S
S
S
H2N Phe Val Asn Gln His Leu Cys Gly Ser His Leu Val Glu Ala Leu Tyr Leu Val Cys Gly Glu Arg Gly Phe Phe Tyr Thr Pro Lys Thr CO2H
B chain
B1
H2N
B29
O
N
O
O
H
N
O
HN Phe

15. The insulin derivative of claim 1, having the formula:

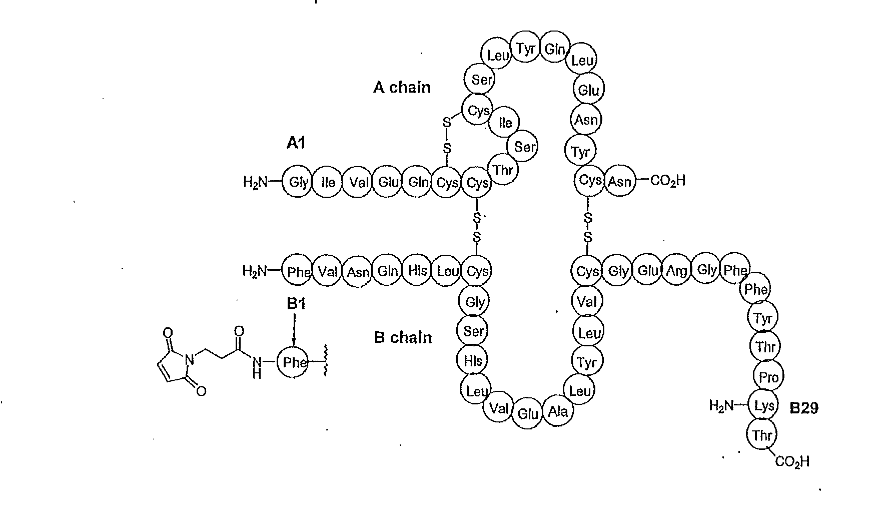
A chain
A1
H2N Gly Ile Val Glu Gln Cys Cys Thr Ser Ile Cys Ser Leu Tyr Gln Leu Glu Asn Tyr Cys Asn CO2H
S
S
S
S
S
S
H2N Phe Val Asn Gln His Leu Cys Gly Ser His Leu Val Glu Ala Leu Tyr Leu Val Cys Gly Glu Arg Gly Phe Phe Tyr Thr Pro Lys Thr CO2H
B chain
B1
O
N
O
O
N
H
Phe
H2N
B29

16. An insulin conjugate comprising an insulin derivative according to any one of claims 1 to 15 and a blood component, wherein the reactive group and the blood component are conjugated through a covalent bond formed between said reactive group and said blood component.

17. The insulin derivative of claim 1, or the insulin conjugate of claim 16, wherein the blood component is a blood protein.

18. The insulin derivative or conjugate of claim 17, wherein the blood protein is serum albumin.

19. The insulin conjugate of claim 16, wherein said conjugate was formed ex vivo.

20. A pharmaceutical composition comprising the insulin derivative of any one of claims 1 to 15 or of claims 17 or 18, or the insulin conjugate of any one of claims 16 to 19, in association with a pharmaceutically acceptable carrier.

21. Use of the insulin derivative of any one of claims 1 to 15 or of claims 17 or 18, or the insulin conjugate of any one of claims 16 to 19, for the preparation of a medicament for the treatment of a glycaemic-related disease or disorder.

22. The use as claimed in claim 21, wherein said glycaemic-related disease is selected from the group consisting of diabetes, diabetes of type I, diabetes of type II, gestational diabetes, cystic fibrosis, polycystic ovary syndrome and pancreatitis.

23. The use as claimed in claim 21, wherein the glycaemic-related disease is selected from the group consisting of diabetes, diabetes of type I and diabetes of type II.
